# EUROPEAN PATENT SPECIFICATION

(11) **EP 3 946 364 B1**
(45) Date of publication and mention of the grant of the patent: **19.11.2025**
(21) Application number: 20782987.0
(22) Date of filing: 01.04.2020
(51) Int. Cl.: A61K 45/06, A61P 31/04, C07F 5/02

(54) **SOLID FORMS OF AN ORALLY-DELIVERED BETA-LACTAMASE INHIBITOR AND USES THEREOF**
FESTE FORMEN EINES ORAL VERABREICHTEN BETA-LACTAMASE-INHIBITORS UND VERWENDUNGEN DAVON
FORMES SOLIDES D'UN INHIBITEUR DE BÊTA-LACTAMASES ADMINISTRÉ PAR VOIE ORALE ET LEURS UTILISATIONS

(30) Priority: 02.04.2019 US 201962828349 P
(43) Date of publication of application: 09.02.2022
(73) Proprietor: Venatorx Pharmaceuticals, Inc., Malvern, PA 19355 (US)
(72) Inventor: BURNS, Christopher J., Malvern, Pennsylvania 19355 (US); ROSEN, Lawrence, Malvern, Pennsylvania 19355 (US); CONDON, Stephen M., Malvern, Pennsylvania 19355 (US); MESAROS, Eugen F., Malvern, Pennsylvania 19355 (US); ZULLI, Allison L., Malvern, Pennsylvania 19355 (US); TROUT, Robert E. Lee, Malvern, Pennsylvania 19355 (US); DENG, Yijun, Malvern, Pennsylvania 19355 (US); BOYD, Steven A., Malvern, Pennsylvania 19355 (US); SIMPSON, Robert, Malvern, Pennsylvania 19355 (US)
(74) Representative: Thwaite, Jonathan Simon
(86) International application number: PCT/US2020/026114
(87) International publication number: WO 2020/205932

(56) References cited:
- WO-A1-2015/191907
- WO-A1-2020/112542
- US-A1- 2010 292 185
- US-A1- 2015 291 630
- US-A1- 2017 342 092

## Description

### BACKGROUND OF THE INVENTION

Antibiotics are the most effective drugs for treating bacteria-infectious diseases. They are largely used in the clinic because of their good antibacterial effect with limited side effects. Among them, the beta-lactam class of antibiotics (for example, penicillins, cephalosporins, monobactams and carbapenems) are preferred because their effect is bactericidal, and their target is absent in eukaryotic cells with consequent low toxicity.

To counter the efficacy of the various beta-lactams, bacteria have evolved to produce variants of beta-lactam deactivating enzymes called beta-lactamases, and in the ability to share this tool both vertically and horizontally inter- and intra-species. These beta-lactamases are categorized as "serine" or "metallo" based, respectively, based on the presence of a key serine or zinc in the enzyme active site. The rapid induction, selection and spread of this mechanism of bacterial resistance can severely limit the whole class of beta-lactam treatment options in the hospital and in the community. There is a need for effective and safe therapeutic agents that can treat such resistant infections.
WO 2020/112542 A1 (04 June 2020) describes pharmaceutical compositions containing boron-containing compounds and their use as inhibitors of beta-lactamase enzymes and as antibacterial agents in combination with a beta-lactam antibiotics. The compounds have the following structure: 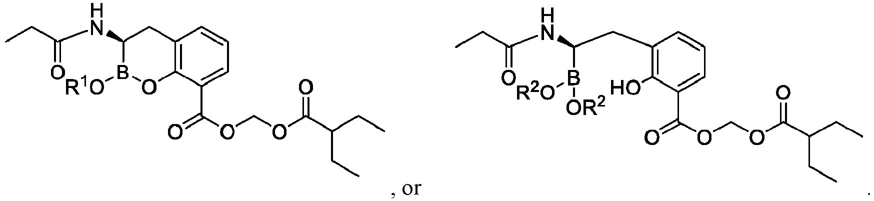 WO 2015/191907 A1 (17 December 2015 ) describes compounds and compositions that modulate the activity of beta-lactamases. The compounds have the following structure:

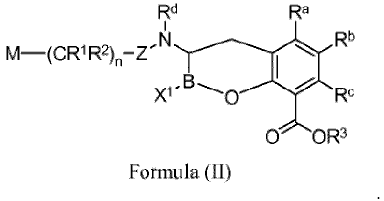

### SUMMARY OF THE INVENTION

Disclosed herein is a crystalline form of the compound: 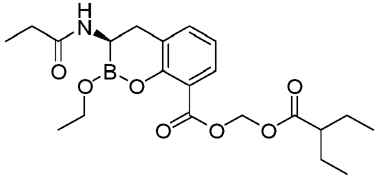 wherein the crystalline form has an X-ray powder diffraction (XRPD) pattern comprising characteristic peaks at 6.1 °±0.1° 20, 9.9 °±0.1° 20, and 16.0 °±0.1° 2θ. In some embodiments, , the X-ray powder diffraction (XRPD) pattern further comprises characteristic peaks at 19.3 °±0.1° 20, 6.8 °±0.1° 20, and 17.9 °±0.1° 2θ. In some embodiments, the X-ray powder diffraction (XRPD) pattern further comprises characteristic peaks at 14.3 °±0.1° 2θ and 21.2 °±0.1° 2θ. In some embodiments, the crystalline form has a DSC thermogram with a broad endotherm having an onset at 112.8 °C.

Also disclosed herein is the crystalline form of the compound: 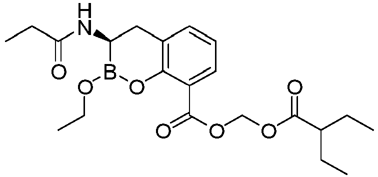 wherein the crystalline form has an X-ray powder diffraction (XRPD) pattern comprising characteristic peaks at 9.3 °±0.1° 20, 12.9 °±0.1° 20, and 21.5 °±0.1° 2θ. In some embodiments, the X-ray powder diffraction (XRPD) pattern further comprises characteristic peaks at 8.8 °±0.1° 20, 14.6 °±0.1° 20, and 17.3 °±0.1° 2θ. In some embodiments, the crystalline form has a DSC thermogram with a broad endotherm having an onset at 116.9 °C.

Also disclosed herein is a pharmaceutical composition comprising a) a crystalline form as described above; and b) a pharmaceutically acceptable excipient. In some embodiments, the pharmaceutical composition further comprises a beta-lactam antibiotic. In some embodiments, the beta-lactam antibiotic is a penicillin, a cephalosporin, a carbapenem, a monobactam, or a combination thereof.

Also disclosed herein is a crystalline form as described above for use in combination with a beta-lactam antibiotic in a method of treating a bacterial infection in a subject. In some embodiments, the beta-lactam antibiotic is ceftibuten, or a salt thereof.

### BRIEF DESCRIPTION OF THE DRAWINGS

**FIG. 1A** shows the ¹H NMR spectrum for Compound 1-ethanolate Form A (in CD₂Cl₂).
**FIG. 1B** shows the ¹³C NMR spectrum for Compound 1-ethanolate Form A (in CD₂Cl₂).
**FIG. 2** shows the FT-IR spectrum for Compound 1-ethanolate Form A.
**FIG. 3** shows the FT-Raman spectrum for Compound 1-ethanolate Form A.
**FIG. 4** shows the XRPD pattern for Compound 1-ethanolate Form A.
**FIG. 5** shows the indexing solution for Compound 1-ethanolate Form A with Cu-Kα radiation.
**FIG. 6** shows the DSC thermogram of Compound 1-ethanolate Form A.
**FIG. 7** shows the DVS analysis of Compound 1-ethanolate Form A (weight% vs. relative humidity).
**FIG. 8** shows an overlay of FT-IR Spectra for Compound 1-ethanolate Forms. Top: Form B; Bottom, Form A.
**FIG. 9A** shows the FT-Raman spectrum for Compound 1-ethanolate Form B.
**FIG. 9B** shows an overlay of Raman Spectra for Compound 1-ethanolate Forms. Top: Form B; Bottom, Form A.
**FIG. 10** shows the indexing solution for Compound 1-ethanolate Form B, with Cu-Kα radiation.
**FIG. 11** shows the DSC thermogram of Compound 1-ethanolate Form B.
**FIG. 12** shows the DVS analysis of Compound 1-ethanolate Form B (weight% vs. relative humidity).
**FIG. 13** shows the x-ray structure of reference Compound 1-methanolate.
**FIG. 14A** shows the optical microscopy image of Compound 1-ethanolate Form A recorded without crossed polarizers. The sample clearly has a needle-like morphology.
**FIG. 14B** shows optical microscopy images of Compound 1-ethanolate Form A recorded with crossed polarizers. The sample clearly has a needle-like morphology.
**FIG. 15A** shows the optical microscopy image of Compound 1-ethanolate Form B recorded without crossed polarizers. The sample generally has a plate-like morphology.
**FIG. 15B** shows optical microscopy images of Compound 1-ethanolate Form B recorded with crossed polarizers. The sample generally has a plate-like morphology.

### DETAILED DESCRIPTION

References to methods of treatment by therapy or surgery or *in vivo* diagnosis methods in this description are to be interpreted as references to compounds, pharmaceutical compositions, and medicaments for use in those methods.

### Reference Compound 1

Reference Compound 1 is ((2-ethylbutanoyl)oxy)methyl (R)-2-hydroxy-3-propionamido-3,4-dihydro-2H-benzo[e] [1,2]oxaborinine-8-carboxylate: 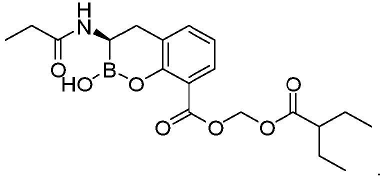 Reference Compound 1 may exist in equilibrium as shown below: 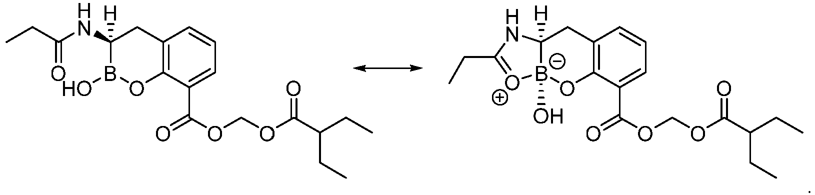 Reference Compound 1 may exist in an equilibrium between the "closed" cyclic form (as shown above) and the "open" acyclic form: 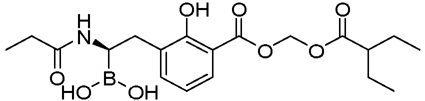 ((R)-(2-(3-((((2-ethylbutanoyl)oxy)methoxy)carbonyl)-2-hydroxyphenyl)-1-propionamidoethyl)boronic acid). Reference Compound 1 may associate into intramolecular dimers, trimers, and any combinations thereof.

### Compound 1-ethanolate

Compound 1-ethanolate is ((2-ethylbutanoyl)oxy)methyl (R)-2-ethoxy-3-propionamido-3,4-dihydro-2H-benzo[e][1,2]oxaborinine-8-carboxylate: 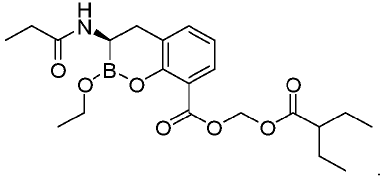 Compound 1-ethanolate may exist in equilibrium as shown below: 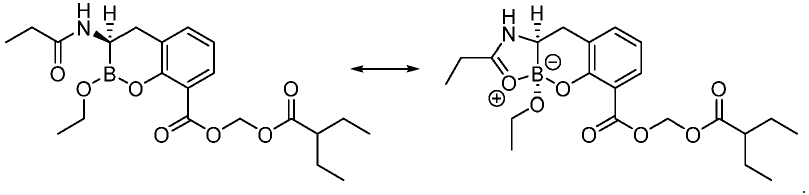 Compound 1-ethanolate may convert to Reference Compound 1 when in contact with water:

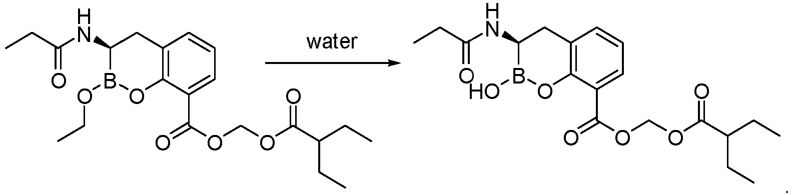

### Reference Compound 1-methanolate

Reference Compound 1 may exist in solid form as a covalently bound solvate. Reference Compound 1 may exist in solid form as a covalently bound methanolate. Reference Compound 1 in solid form may be ((2-ethylbutanoyl)oxy)methyl (R)-2-methoxy-3-propionamido-3,4-dihydro-2H-benzo[e][1,2]oxaborinine-8-carboxylate: 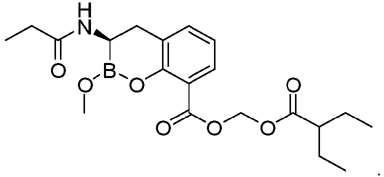 Reference Compound 1-methanolate may exist in equilibrium as shown below: 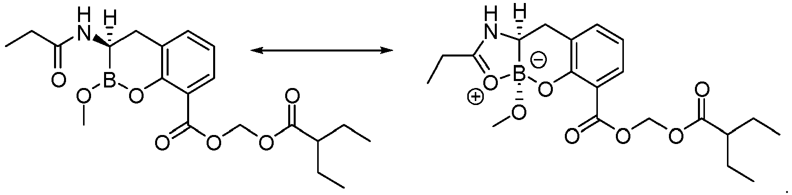 Reference Compound 1-methanolate may convert to Reference Compound 1 when in contact with water:

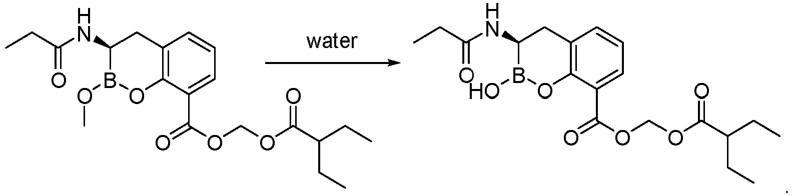

### Polymorph Forms

Disclosed herein are crystalline polymorph Forms A and B of ((2-ethylbutanoyl)oxy)methyl (R)-2-ethoxy-3-propionamido-3,4-dihydro-2H-benzo[e][1,2]oxaborinine-8-carboxylate.

Also described herein are processes for the preparation of the crystalline polymorph Forms A and B.

### Polymorph Form A

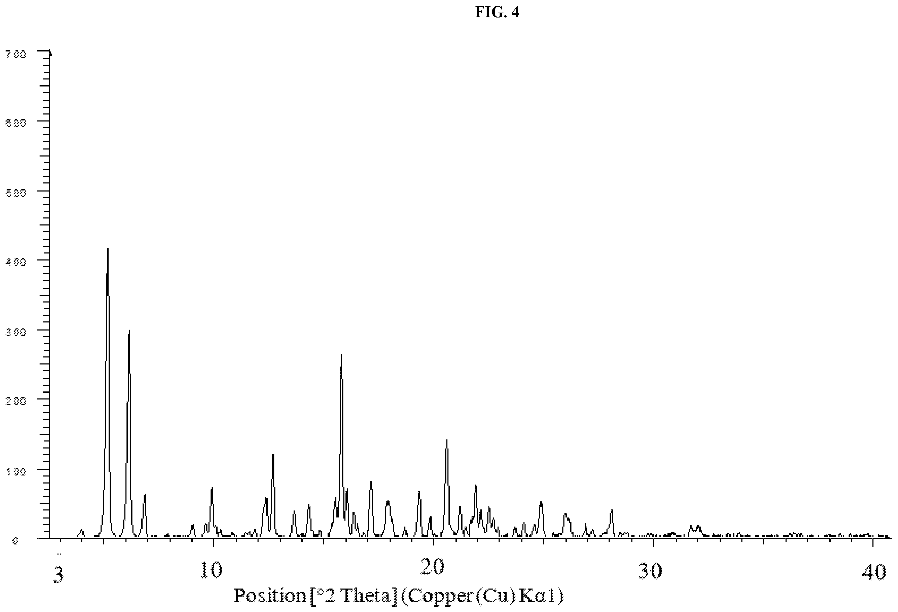

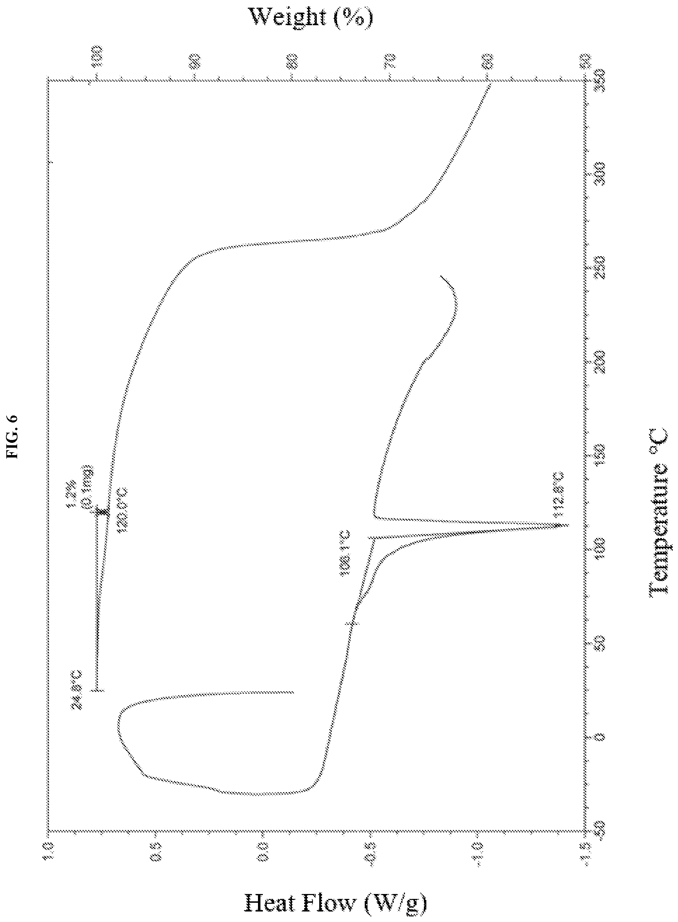

The term "polymorph Form A" or "Form A" refers to a crystalline form of ((2-ethylbutanoyl)oxy)methyl (R)-2-ethoxy-3-propionamido-3,4-dihydro-2H-benzo[e][1,2]oxaborinine-8-carboxylate that exhibits an X-ray powder diffraction pattern substantially the same as that shown in FIG. 4 and/or a DSC thermogram substantially the same as that shown in FIG. 6. In some embodiments, a polymorph of ((2-ethylbutanoyl)oxy)methyl (R)-2-ethoxy-3-propionamido-3,4-dihydro-2H-benzo[e] [1,2]oxaborinine-8-carboxylate is characterized by the major peaks of FIG. 4. In some embodiments, the major peaks are the peaks of at least 20%, at least 15% or at least 10% of maximum intensity in the XRPD pattern of FIG. 4.

In one embodiment, ((2-ethylbutanoyl)oxy)methyl (R)-2-ethoxy-3-propionamido-3,4-dihydro-2H-benzo[e][1,2]oxaborinine-8-carboxylate polymorph Form A exhibits an X-ray powder diffraction pattern characterized by the diffraction pattern summarized in Table 1. In some embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate comprises at least 3 peaks of (±0.1°2θ) of Table 1. In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate comprises at least 4 peaks of (±0.1°2θ) of Table 1, at least 5 peaks of (±0.1°2θ) of Table 1, at least 6 peaks of (±0.1°2θ) of Table 1, at least 7 peaks of (±0.1'2θ) of Table 1, at least 8 peaks of (±0.1°2θ) of Table 1, or at least 9 peaks of (±0.1°2θ) of Table 1.

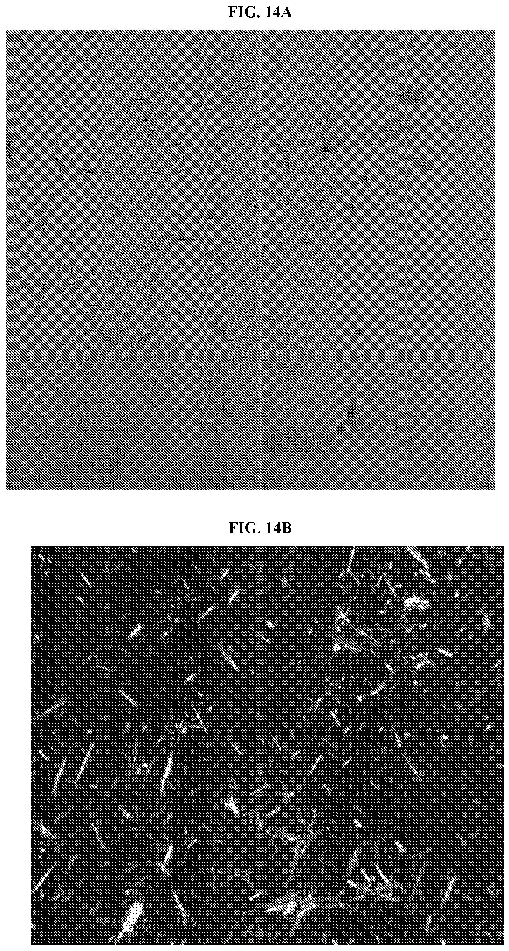

Polymorph form A crystalizes as needles (FIG. 14A and FIG. 14B) and is thermodynamically stable. In some embodiments, polymorph Form A is more thermodynamically stable than polymorph Form B. In some embodiments, polymorph Form A is less dense than polymorph Form B. In some embodiments, a less dense polymorphic form is preferred for formulation purposes.

**Table 1. Form A Characteristic XRPD Signals (2θ, Cu)**

| **Angle 2-Theta °** | **Intensity, normalized** | **d-value, Ångstrom** |
|---|---|---|
| 3.95 | 2.7 | 22.3567 |
| 5.14 | 100.0 | 17.1938 |
| 6.11 | 71.8 | 14.4653 |
| 6.81 | 14.9 | 12.9761 |
| 9.00 | 4.3 | 9.8237 |
| 9.58 | 5.0 | 9.2208 |
| 9.89 | 17.4 | 8.9401 |
| 12.23 | 10.9 | 7.2318 |
| 12.35 | 13.8 | 7.1636 |
| 12.66 | 28.7 | 6.9892 |
| 13.62 | 8.8 | 6.4982 |
| 14.29 | 11.4 | 6.1922 |
| 15.52 | 13.8 | 5.7067 |
| 15.78 | 63.0 | 5.6111 |
| 16.02 | 16.7 | 5.5281 |
| 16.33 | 8.9 | 5.4233 |
| 16.51 | 4.8 | 5.3641 |
| 17.14 | 19.4 | 5.1686 |
| 17.87 | 12.3 | 4.9588 |
| 18.10 | 6.5 | 4.8967 |
| 19.34 | 16.0 | 4.5865 |
| 19.84 | 7.2 | 4.4706 |
| 20.59 | 33.8 | 4.3105 |
| 21.19 | 10.8 | 4.1890 |
| 21.91 | 18.0 | 4.0542 |
| 22.14 | 9.3 | 4.0127 |
| 22.52 | 10.6 | 3.9458 |

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate comprises characteristic peaks at 5.1 °±0.1° 20, 6.1 °±0.1° 20, and 15.8 °±0.1° 2θ.

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate further comprises characteristic peaks at 12.7 °±0.1° 20, 17.1 °±0.1° 20, and 20.6 °±0.1° 2θ.

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate comprises characteristic peaks at 6.1 °±0.1° 20, 9.9 °±0.1° 20, and 16.0 °±0.1° 2θ.

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate further comprises characteristic peaks at 19.3 °±0.1° 20, 6.8 °±0.1° 20, and 17.9 °±0.1° 2θ.

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate further comprises characteristic peaks at 14.3 °±0.1° 2θ and 21.2 °±0.1° 2θ.

In certain embodiments, the polymorph Form A of ((2-ethylbutanoyl)oxy)methyl (R)-2-ethoxy-3-propionamido-3,4-dihydro-2H-benzo[e][1,2]oxaborinine-8-carboxylate has a broad endotherm having an onset at 112.8 °C.

### Polymorph Form B

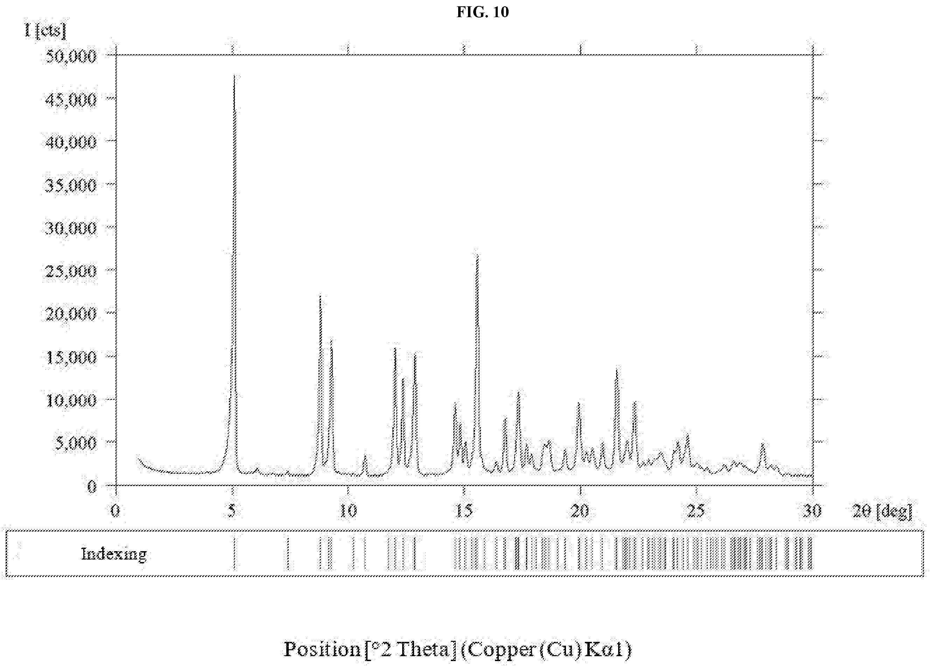

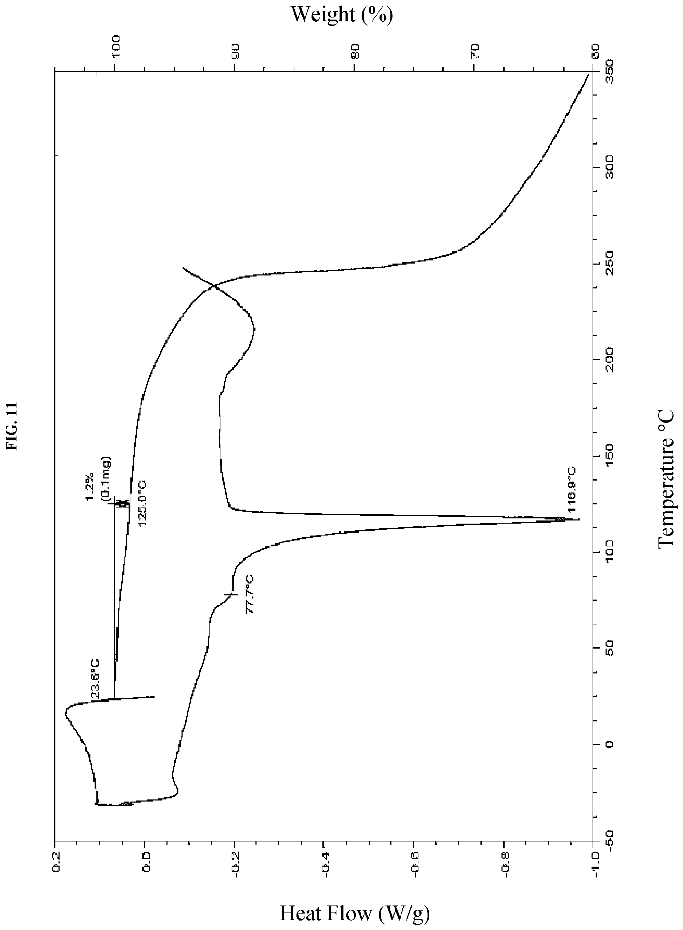

The term "polymorph Form B" or "Form B" or refers to a crystalline form of ((2-ethylbutanoyl)oxy)methyl (R)-2-ethoxy-3-propionamido-3,4-dihydro-2H-benzo[e][1,2]oxaborinine-8-carboxylate that exhibits an X-ray powder diffraction pattern substantially the same as that shown in FIG. 10, and/or a DSC thermogram substantially the same as that shown in FIG. 11.

In one embodiment, ((2-ethylbutanoyl)oxy)methyl (R)-2-ethoxy-3-propionamido-3,4-dihydro-2H-benzo[e][1,2]oxaborinine-8-carboxylate polymorph Form B exhibits an X-ray powder diffraction pattern characterized by the diffraction pattern summarized in Table 2. In some embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate comprises at least 3 peaks of (±0.1°2θ) of Table 2. In certain embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate comprises at least 4 peaks of (±0.1°2θ) of Table 2, at least 5 peaks of (±0.1°2θ) of Table 2, at least 6 peaks of (±0.1°2θ) of Table 2, at least 7 peaks of (±0.1°2θ) of Table 2, at least 8 peaks of (±0.1°2θ) of Table 2, or at least 9 peaks of (±0.1°2θ) of Table 2.

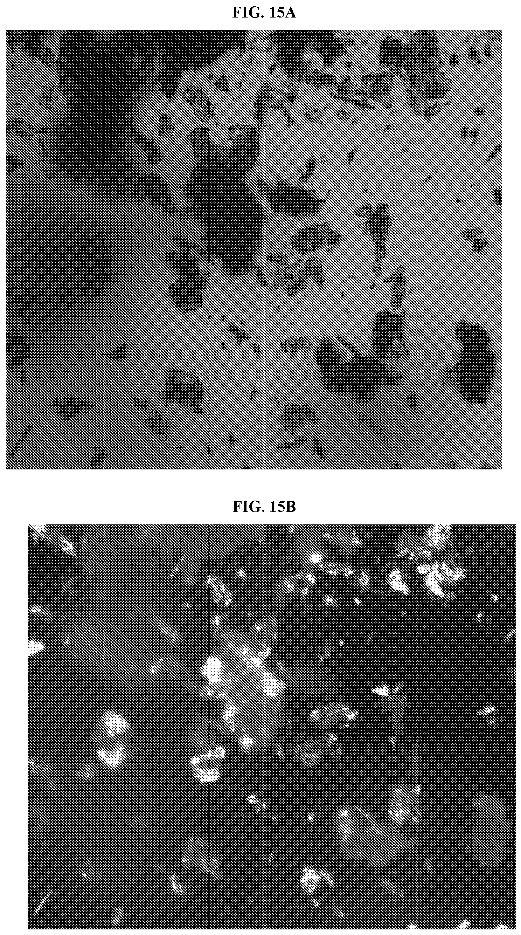

Polymorph Form B crystallizes as large plates (FIG. 15A and FIG. 15B). In some embodiments, large plates are easier to handle and purify.

**Table 2. Form B Characteristic XRPD Signals (2θ, Cu Kα1)**

| **Angle 2-Theta °** | **Intensity, normalized** | **d-value, Ångstrom** |
|---|---|---|
| 5.10 | 100.0 | 17.3010 |
| 8.80 | 10.5 | 10.0414 |
| 9.29 | 20.8 | 9.5172 |
| 12.02 | 11.1 | 7.3557 |
| 12.35 | 13.6 | 7.1589 |
| 12.86 | 21.6 | 6.8783 |
| 14.60 | 10.5 | 6.0637 |
| 15.06 | 5.4 | 5.8780 |
| 15.34 | 7.0 | 5.7702 |
| 15.56 | 39.5 | 5.6923 |
| 16.76 | 4.1 | 5.2858 |
| 17.33 | 15.0 | 5.1121 |
| 18.48 | 5.2 | 4.7966 |
| 18.64 | 7.4 | 4.7565 |
| 19.92 | 12.2 | 4.4536 |
| 20.25 | 3.2 | 4.3819 |
| 20.50 | 21.4 | 4.3280 |
| 20.92 | 4.0 | 4.2429 |
| 21.54 | 17.4 | 4.1222 |
| 21.97 | 5.8 | 4.0429 |
| 22.31 | 7.9 | 3.9810 |
| 23.49 | 5.0 | 3.7846 |
| 24.60 | 6.5 | 3.6166 |
| 27.84 | 4.5 | 3.2016 |

In certain embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate comprises characteristic peaks at 5.1 °±0.1° 20, 12.9 °±0.1° 20, and 15.6 °±0.1° 2θ.

In certain embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate further comprises characteristic peaks at 9.3 °±0.1° 20, 20.5 °±0.1° 20, and 21.5 °±0.1° 2θ.

In certain embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate comprises characteristic peaks at 9.3 °±0.1° 20, 12.9 °±0.1° 20, and 21.5 °±0.1° 2θ.

In certain embodiments, the polymorph Form B of ((2-ethylbutanoyl)oxy)methyl (R)-2-ethoxy-3-propionamido-3,4-dihydro-2H-benzo[e][1,2]oxaborinine-8-carboxylate further comprises characteristic peaks at 8.8 °±0.1° 20, 14.6 °±0.1° 20, and 17.3 °±0.1° 2θ.

### Methods of Treatment

References to methods of treatment by therapy or surgery or *in vivo* diagnosis methods in this description are to be interpreted as references to compounds, pharmaceutical compositions, and medicaments for use in those methods.

The present disclosure also describes methods for inhibiting bacterial growth, by, e.g., reducing bacterial resistance to a β-lactam antibiotic, such methods comprising contacting a bacterial cell culture, or a bacterially infected cell culture, tissue, or organism, with Compound 1-ethanolate. The bacteria to be inhibited by administration of Compound 1-ethanolate may be bacteria that are resistant to beta-lactam antibiotics. The term "resistant" is well-understood by those of ordinary skill in the art (see, e g Payne et al., Antimicrobial Agents and Chemotherapy 38 767-772 (1994), Hanaki et al., Antimicrobial Agents and Chemotherapy 30 1120-1126 (1995)).

These methods are useful for inhibiting bacterial growth in a variety of contexts. Compound 1-ethanolate may be administered to an experimental cell culture *in vitro* to prevent the growth of beta-lactam resistant bacteria. In certain other instances, Compound 1-ethanolate is administered to a mammal, including a human to prevent the growth of beta-lactam resistant bacteria *in vivo.* The method according to this instance comprises administering a therapeutically effective amount of a beta-lactamase inhibitor for a therapeutically effective period of time to a mammal, including a human. Preferably, the beta-lactamase inhibitor is administered in the form of a pharmaceutical composition as described above. An antibiotic may be co-administered with the beta-lactamase inhibitor. The antibiotic may be a beta-lactam antibiotic. The beta-lactam antibiotic may be ceftibuten, or a salt thereof. The beta-lactam antibiotic is cefixime, or a salt thereof.

Also described herein are methods of treating a bacterial infection, which method comprises administering to a subject a pharmaceutical composition comprising Compound 1-ethanolate and a pharmaceutically acceptable excipient as described above. The bacterial infection may be an upper or lower respiratory tract infection, a urinary tract infection, an intra-abdominal infection, or a skin infection.

The infection that is treated or prevented may comprise a bacteria that includes *Elizabethkingia meningoseptica, Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides* 3452A homology group, *Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus* subsp. *hyicus, Staphylococcus haemolyticus, Staphylococcus hominis,* or *Staphylococcus saccharolyticus.*

The infection that is treated or prevented may comprise a bacteria that includes *Elizabethkingia meningoseptica , Pseudomonas aeruginosa, Pseudomonas fluorescens, Stenotrophomonas maltophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Acinetobacter calcoaceticus, Acinetobacter haemolyticus, Yersinia enterocolitica, Yersinia pestis, Yersinia pseudotuberculosis, Yersinia intermedia, Haemophilus influenzae, Haemophilus parainfluenzae, Haemophilus haemolyticus, Haemophilus parahaemolyticus, Helicobacter pylori, Campylobacter fetus, Campylobacter jejuni, Campylobacter coli, Vibrio cholerae, Vibrio parahaemolyticus, Legionella pneumophila, Listeria monocytogenes, Neisseria gonorrhoeae, Neisseria meningitidis, Moraxella, Bacteroides fragilis, Bacteroides vulgatus, Bacteroides ovalus, Bacteroides thetaiotaomicron, Bacteroides uniformis, Bacteroides eggerthii,* or *Bacteroides splanchnicus.*

### Certain Definitions

Unless defined otherwise, all technical and scientific terms used herein have the same meanings as commonly understood by one of ordinary skill in the art. Although any methods and materials similar or equivalent to those described herein can be used in the practice or testing of embodiments described herein, certain preferred methods, devices, and materials are now described.

As used herein and in the appended claims, the singular forms "a," "an," and "the" include plural reference unless the context clearly dictates otherwise. Thus, for example, reference to "an excipient" is a reference to one or more excipients and equivalents thereof known to those skilled in the art, and so forth.

The term "about" is used to indicate that a value includes the standard level of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and to "and/or."

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

"Optional" or "optionally" may be taken to mean that the subsequently described structure, event or circumstance may or may not occur, and that the description includes instances where the events occurs and instances where it does not.

As used in the specification and appended claims, unless specified to the contrary, the following terms have the meaning indicated below.

The term "animal" as used herein includes, but is not limited to, humans and non-human vertebrates such as wild, domestic and farm animals. As used herein, the terms "patient," "subject" and "individual" are intended to include living organisms in which certain conditions as described herein can occur. Examples include humans, monkeys, cows, sheep, goats, dogs, cats, mice, rats, and transgenic species thereof. In a preferred embodiment, the patient is a primate. In certain embodiments, the primate or subject is a human. In certain instances, the human is an adult. In certain instances, the human is child. In further instances, the human is 12 years of age or younger. In certain instances, the human is elderly. In other instances, the human is 60 years of age or older. Other examples of subjects include experimental animals such as mice, rats, dogs, cats, goats, sheep, pigs, and cows.

By "pharmaceutically acceptable," it is meant the carrier, diluent or excipient must be compatible with the other ingredients of the composition and not deleterious to the recipient thereof.

The term "pharmaceutical composition" means a composition comprising at least one active ingredient, such as Compound 1-ethanolate, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

A "therapeutically effective amount" or "effective amount" as used herein refers to the amount of active compound or pharmaceutical agent that elicits a biological or medicinal response in a tissue, system, animal, individual or human that is being sought by a researcher, veterinarian, medical doctor or other clinician, which includes one or more of the following: (1) preventing the disease; for example, preventing a disease, condition or disorder in an individual that may be predisposed to the disease, condition or disorder but does not yet experience or display the pathology or symptomatology of the disease, (2) inhibiting the disease; for example, inhibiting a disease, condition or disorder in an individual that is experiencing or displaying the pathology or symptomatology of the disease, condition or disorder (i.e., arresting further development of the pathology and/or symptomatology), and (3) ameliorating the disease; for example, ameliorating a disease, condition or disorder in an individual that is experiencing or displaying the pathology or symptomatology of the disease, condition or disorder (i.e., reversing the pathology and/or symptomatology).

The terms "treat," "treated," "treatment," or "treating" as used herein refers to both therapeutic treatment in some embodiments and prophylactic or preventative measures in other embodiments, wherein the object is to prevent or slow (lessen) an undesired physiological condition, disorder or disease, or to obtain beneficial or desired clinical results. For the purposes described herein, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment. A prophylactic benefit of treatment includes prevention of a condition, retarding the progress of a condition, stabilization of a condition, or decreasing the likelihood of occurrence of a condition. As used herein, "treat," "treated," "treatment," or "treating" includes prophylaxis in some embodiments.

The term "substantially the same as" as used herein, refers to a powder x-ray diffraction pattern or differential scanning calorimetry pattern that is non-identical to those depicted herein, but that falls within the limits of experimental error, when considered by one of ordinary skill in the art.

### EXAMPLES

### Analytical Methods

### High-resolution Mass Spectrometry (HRMS)

High resolution mass spectra from 50 to 3000 Da were collected with a Bruker Maxis-Plus QTOF mass spectrometer using an electrospray ionization source. The instrument was controlled and data analyzed using Bruker Compass v.4.4 software. The mass spectrometer was calibrated immediately prior to analyses and was operated in positive ionization mode using an Electrospray Ionization (ESI) source. Samples were prepared by dilution in absolute ethanol (without denaturants) or HPLC grade water prior to direct infusion into the ion source.

### Raman Spectroscopy

Raman spectra were acquired on a Bruker MultiRAM with OPUS 7.0 software. The samples were measured using truncated NMR tubes that were filled in a N2-filled glovebox at approximately 0% RH. A 300 mW laser power from a Nd:YAG laser (1064 nm excitation wavelength) was used to irradiate the sample. Each spectrum represents 64 co-added scans collected at a spectral resolution of 2 cm⁻¹.

### FT-IR Spectroscopy (FT-IR)

Infrared spectra were acquired using a Nicolet 6700 Fourier transform infrared (FT-IR) spectrophotometer (Thermo Nicolet) equipped with an Ever-Glo mid/far IR source, an extended range potassium bromide (KBr) beam splitter, and a deuterated triglycine sulfate (DTGS)) detector. Wavelength verification was performed using NIST SRM 1921b (polystyrene). An attenuated total reflectance (ATR) accessory (Thunderdome^{™}, Thermo Spectra-Tech) equipped with a germanium (Ge) crystal was used for data acquisition. Each sample was placed directly on the clean Ge crystal for analysis. Each spectrum represents 256 co-added scans collected at a spectral resolution of 4 cm⁻¹. A background data set was acquired with a clean Ge crystal.

### X-ray powder diffraction

XRPD patterns were collected with two conditions. A PANalytical X'Pert PRO MPD diffractometer using an incident beam of Cu Kα radiation produced using a long, fine-focus source and a nickel filter. An elliptically graded multilayer mirror was used to focus Cu Kα X-rays through the specimen and onto the detector. Data were collected and analyzed using Data Collector software v. 2.2b. Prior to the analysis, a silicon specimen (NIST SRM 640e) was analyzed to verify the observed position of the Si 111 peak was consistent with the NIST-certified position. A specimen of the sample was sandwiched between 3-µm-thick films and analyzed in transmission geometry. Anti-scatter slits (SS) were used to minimize the background generated by air. Soller slits for the incident and diffracted beams were used to minimize broadening from axial divergence. Diffraction patterns were collected using a scanning position-sensitive detector (X'Celerator) located 240 mm from the sample and Data Collector software v. 2.2b. The data acquisition parameters for each pattern are displayed above the image in the Data section of this report including the divergence slit (DS).

A second method used a Bruker D8 Advance using Cu Kα radiation of 40 kV/40 mA. Data were collected with a LynxEye detector in Bragg-Brentano reflection geometry with a 0.02°2q step size, 37 s step time over 2.5-50°2q range. The powder samples were measured in 0.05 mm deep silicon single-crystal sample holders covered with a Kapton foil to protect them from moisture. The samples were placed in an inert atmosphere environment (N₂-filled glovebox) but no other special treatment was used in preparing the samples other than the application of slight pressure to obtain a flat surface. All samples were rotated during the measurement.

### Indexing

XRPD patterns were indexed using TRIADS3. Indexing and structure refinement are computational studies. Agreement between the allowed peak positions, marked with red bars, and the observed peaks indicates a consistent unit cell determination. Successful indexing of the pattern indicates that the sample is composed primarily of a single crystalline phase. Space groups consistent with the assigned extinction symbol, unit cell parameters, and derived quantities are tabulated below each figure showing tentative indexing solution. To confirm the tentative indexing solution, the molecular packing motifs within the crystallographic unit cells must be determined. No attempts at molecular packing were performed.

### Polarized Light Microscopy (PLM)

Polarized light microscopy was performed using a Leica MZ12.5 or Fisher Scientific Stereomaster stereomicroscope. Samples were observed using 0.8-10x objectives with crossed polarizers.

### Differential scanning calorimetry (DSC) analysis

DSC was performed using a TA Instruments 2920 or Q2000 differential scanning calorimeter equipped with a refrigerated cooling system (RCS). Temperature calibration was performed using NIST-traceable indium metal. The sample was placed into an aluminum DSC pan, covered with a lid, and the weight was accurately recorded. A weighed aluminum pan configured as the sample pan was placed on the reference side of the cell. The data acquisition parameters and pan configuration for each thermogram are captured for each analysis. The method code on the thermogram is an abbreviation for the start and end temperature as well as the heating rate; e.g., -30-250-10 means "from -30 °C to 250 °C, at 10 °C/min".

### Dynamic vapor sorption (DVS) analysis

Moisture sorption/desorption data were collected on a VTI SGA-100 Vapor Sorption Analyzer. NaCl and PVP were used as calibration standards. Samples were not dried prior to analysis. Sorption and desorption data were collected over a range from 5% to 95% RH at 10% RH increments under a nitrogen purge (RH = relative humidity). The equilibrium criterion used for analysis was less than 0.0100% weight change in 5 minutes with a maximum equilibration time of 3 hours. Data were not corrected for the initial moisture content of the samples.

A second set of DVS experiments were conducted using a stepped method from 0% RH to 60% RH with steps of 10% RH and four hours of equilibration at each humidity value. These data were collected on a Project Messtechnik (now ProUmid) SPS11-100n. The samples were placed on an aluminum holder and allowed to equilibrate at 0% RH before starting the following predefined humidity program: hold for 4 h at 0% RH, increase humidity by a step of 10% RH, hold for 4 h, repeat the above two steps five times until a value of 60% RH has been reached. The samples were then analyzed for crystallinity using the second set of XRPD conditions.

### Hot Stage Microscopy (HSM)

Hot stage microscopy was performed using a Linkam hot stage (FTIR 600) mounted on a Leica DM LP microscope equipped with a SPOT Insight^{™} color digital camera. Temperature calibrations were performed using USP melting point standards. Samples were placed on a cover glass, and a second cover glass was placed on top of the sample. As the stage was heated, each sample was visually observed using a 20x objective with crossed polarizers and a first order red compensator. Images were captured using SPOT software (v. 4.5.9).

### Thermogravimetric Analysis (TGA)

TGA was performed using a TA Instruments Q5000 IR thermogravimetric analyzer. Temperature calibration was performed using nickel and Alumel^{™}. The sample was placed in an aluminum pan. The sample was hermetically sealed, the lid pierced, then inserted into the TG furnace. The furnace was heated under nitrogen. The data acquisition parameters for each thermogram are displayed in the data image. The method code on the thermogram is an abbreviation for the start and end temperature as well as the heating rate; e.g., 00-350-10 means "from ambient to 350 °C, at 10 °C/min".

### Example 1: Preparation and Characterization of ((2-ethylbutanoyl)oxy)methyl (R)-2-ethoxy-3-propionamido-3,4-dihydro-2H-benzo[e][1,2]oxaborinine-8-carboxylate (Compound 1-ethanolate), Form A.

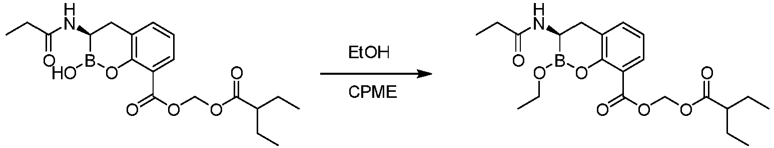

A solution of ((2-ethylbutanoyl)oxy)methyl (*R*)-2-hydroxy-3-propionamido-3,4-dihydro-2*H*-benzo[*e*][1,2]oxaborinine-8-carboxylate (Compound 1) in 18 volumes of cyclopentyl methyl ether (CPME) was distilled under vacuum to until the volume was reduced to 7.5 volumes while maintaining a temperature below ≤65 °C. A Karl Fischer titration was performed to ensure the level of residual water was reduced to a value below 5000 ppm. The solution was diluted to 17 volumes with additional CPME followed by 6 volumes of ethanol. The solution was distilled under vacuum until a volume of ~7.5 volumes was achieved. The composition of the solution was examined to ensure that the level of residual water was below 1000 ppm by KF and the ethanol content was between 6 and 11 volume% determined using ¹H NMR spectroscopy. The temperature was raised to 55 ± 5 °C over 43 min. and methylcyclohexane (22.5 volumes) added over 50 min while maintaining the temperature at 55 ± 5 °C. The temperature was adjusted to 45 ± 5 °C and the mixture seeded with 0.15% by weight ((2-ethylbutanoyl)oxy)methyl (R)-2-ethoxy-3-propionamido-3,4-dihydro-2H-benzo[e][1,2]oxaborinine-8-carboxylate seeds Form A. After addition of the seeds solid formation was observed. The slurry was stirred at 45 ± 5 °C for approximately 4 h, then cooled to 20 ± 5 °C over ~2.5 h and stirred for an additional 11 h at 20 ± 5 °C. The product was isolated by filtration under a nitrogen. The solid was washed with methylcyclohexane (7 volumes) over approximately a 7 h period. The resulting wet cake was dried at 30 °C under vacuum to constant weight over about 68 h. The above process provided ((2-ethylbutanoyl)oxy)methyl (R)-2-ethoxy-3-propionamido-3,4-dihydro-2H-benzo[e][1,2]oxaborinine-8-carboxylate, Form A, in 79% with a purity of 99.7 AUC% by UHPLC.

### Elemental Analysis

Elemental analysis of Compound 1-ethanolate Form A was conducted (Intertek USA, Inc. QTI Whitehouse Station, NJ, USA). Carbon, hydrogen and nitrogen were determined by an elemental analyzer (Perkin-Elmer 2400 Elemental Analyzer) and the data are presented in Table 3.

**Table 3. Elemental Analysis of Compound 1-ethanolate Form A**

| **Element** | **% Theoretical** | **% Observed** |
|---|---|---|
| Boron | 2.65 | 2.86 |
| Carbon | 60.16 | 59.62 |
| Hydrogen | 7.21 | 7.20 |
| Nitrogen | 3.34 | 3.31 |

### Mass Spectrometry

The mass spectrum of Compound 1-ethanolate Form A was obtained using a Waters Q-Tof (quadrupole-time of flight hybrid) micromass spectrometer operating in Electrospray Ionization (ESI) positive ion polarity mode. The sample was prepared at a concentration of approximately 2 µg/mL in absolute ethanol, and infused directly into the mass spectrometer source and the tuning parameters were optimized to the compound.

The mass spectrum included peaks with *m*/*z* 442.21 for the [M+Na]⁺, and *m*/*z* 861.43 for the [2M+Na]⁺ peak, in agreement with the monoisotopic mass of the proposed molecular formula of C₂₁H₃₀BNO₇. The molecular mass of Compound 1-ethanolate Form A is 419.28 Da and the exact mass is 419.21 Da.

### Nuclear Magnetic Resonance (NMR) Spectroscopy

Compound 1-ethanolate Form A was dissolved in 99.8% dichloromethane-*d*₂ (CD₂Cl₂, 0.8 mL) containing 0.05% (v/v) tetramethylsilane (TMS), as the solvent. To minimize moisture exposure, the sample was prepared under a dry N₂ environment. All NMR data were collected at 300K using a Bruker-Biospin 5 mm gradient broadband probe on a Bruker Biospin AVANCE 500 MHz NMR spectrometer. The 1D proton and carbon-13 spectra were acquired at 500 MHz and 125 MHz, respectively. The spectra were referenced using the tetramethylsilane resonance and set equal to 0.0 ppm for ¹H and ¹³C.

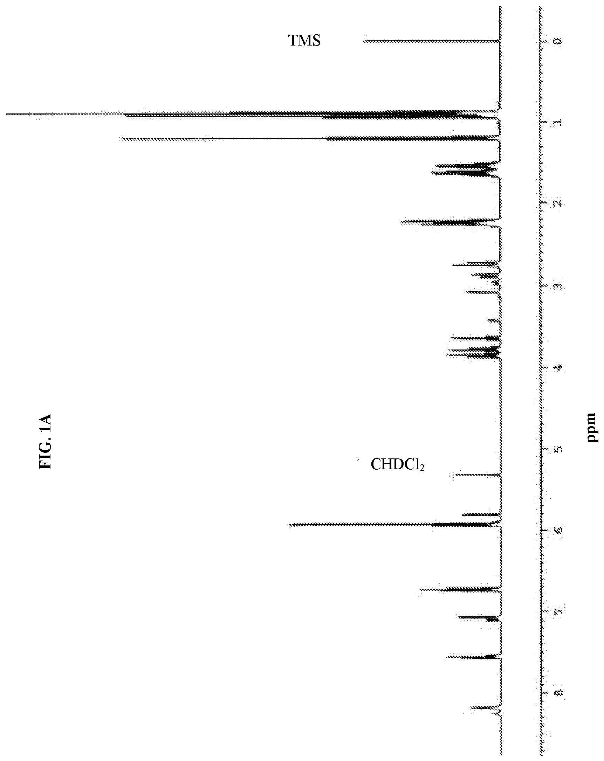

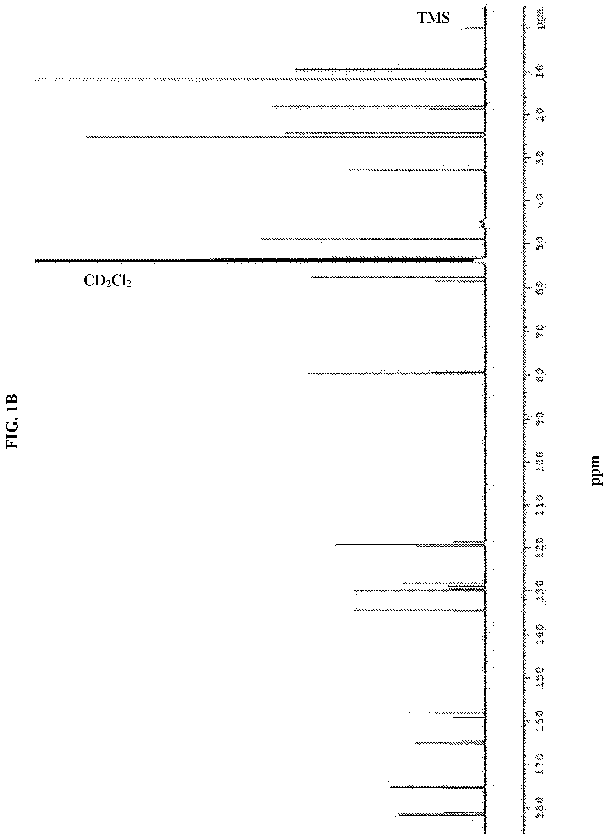

The 1D proton spectrum (FIG. 1A) showed the expected chemical shifts, multiplicities and integrations that are consistent with the structure of Compound 1-ethanolate. However, due to presence of residual water in either the CD₂Cl₂ solvent and/or the Compound 1-ethanolate Form A sample, and/or possibly absorption of moisture from the environment, the products of Compound 1-ethanolate Form A hydrolysis, i.e., ((2-ethylbutanoyl)oxy)methyl (*R*)-2-hydroxy-3-propionamido-3,4-dihydro-2*H*-benzo[*e*][1,2]oxaborinine-8-carboxylate and free (unbound) ethanol, were also observed as minor resonances. The observed chemical shifts from the 1D carbon-13 spectrum (FIG. 1B) were consistent with the described chemical structure of Compound 1-ethanolate. Duplicate ¹H and ¹³C resonances were observed (due to the presence of amide bond rotamers).

**Table 4: ¹H and ¹³C NMR Chemical Shift Assignments for Compound 1-ethanolate Form A in CD₂Cl₂ [300K].**

| 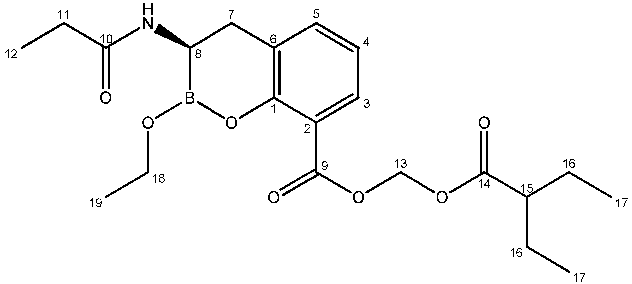 | | | | |
|---|---|---|---|---|
| **Position No.** | **Proton Shift^{#} (ppm)** | **Integration** | **Proton Multiplicity (*J* in Hz)** | **Carbon Shift^{#} (ppm)** |
| 1 | NA | NA | NA | 159.2, 158.3 |
| 2 | NA | NA | NA | 119.7^{¥}, 118.8^{¥} |
| 3 | 7.58 | 1 | m | 129.6 |
| | 7.56 | | m | 130.0 |
| | 7.54 | | m | 129.8 |
| 4 | 6.73 | 1 | m | 119.3^{¥}, 119.2^{¥}, 119.1^{¥} |
| 5 | 7.11 | 1 | bdt (*J =* 7.2) 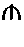 | 134.6, 134.5, 134.4 |
| | 7.07 | | bdt (*J* = 7.2) 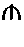 | |
| 6 | NA | NA | NA | 128.9, 128.3 |
| 7 | 2.98 | 1 | dd (*J =* 15.4, 4.3) | 32.9 |
| | 2.89 | | dd (*J =* 15.5, 4.5) | |
| | 2.75 | 1 | dd (*J =* 15.6, 2.1) | |
| 8 | 3.43 | 0.25 | bs | 46.2, 45.0 |
| | 3.13 | 0.05 | bs | |
| | 3.08 | 0.70 | bs | |
| 9 | NA | NA | NA | 165.2, 165.1, 164.6 |
| 10 | NA | NA | NA | 181.6, 181.5, 181.1 |
| 11 | 2.24 | 3* | m | 24.4, 24.3 |
| 12 | 0.92 | 9^{†} | m | 9.6, 9.5 |
| 13 | 5.94 | 1.76 | m | 79.6, 79.5, 79.3 |
| | 5.81 | 0.27 | d (*J =* 5.6) | |
| 14 | NA | NA | NA | 175.5, 175.2 |
| 15 | 2.26 | 3* | m | 49.0 |
| 16 | 1.63 | 4.1 | m | 25.2, 25.1 |
| | 1.54 | | m | |
| 17 | 0.89 | 9^{†} | m | 11.8 |
| 18 | 3.87 | 0.7 | m | 57.6 |
| | 3.79 | 0.7 | m | 57.6 |
| 19 | 1.20 | 2.5^{Δ} | t (*J* = 7.0) | 18.2 |
| 8-NH-10 | 8.47 | 0.04 | bs | NA |
| | 8.25,8.18 | 0.94 | bs | NA |
| CH₂ of Free EtOH | 3.66 | 0.3 | q (*J =* 7.0) | 58.6 |
| CH₃ of Free EtOH | 1.19 | 2.5^{Δ} | t (*J* = 6.9) | 18.7 |

| | | | | |
|---|---|---|---|---|
| bdt = broad doublet of triplets, bs = broad singlet, d = doublet, dd = doublet of doublets, m = multiplet, t = triplet, ppm = parts per million. ^{#}The splitting of several ¹H and ¹³C resonances is most likely due to the presence of rotamers and/or hydrolysis product. NA = Not Applicable. ^{¥}Assignments can be interchanged. 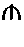 Pair of broad triplets (not resolved completely). *H11 and H15 overlap (total of 3 protons). ^{†}H12 and H17 overlap (total of 9 protons). ^{Δ}H19 overlaps with CH₃ peak of free ethanol. | | | | |

### Infrared Spectroscopy

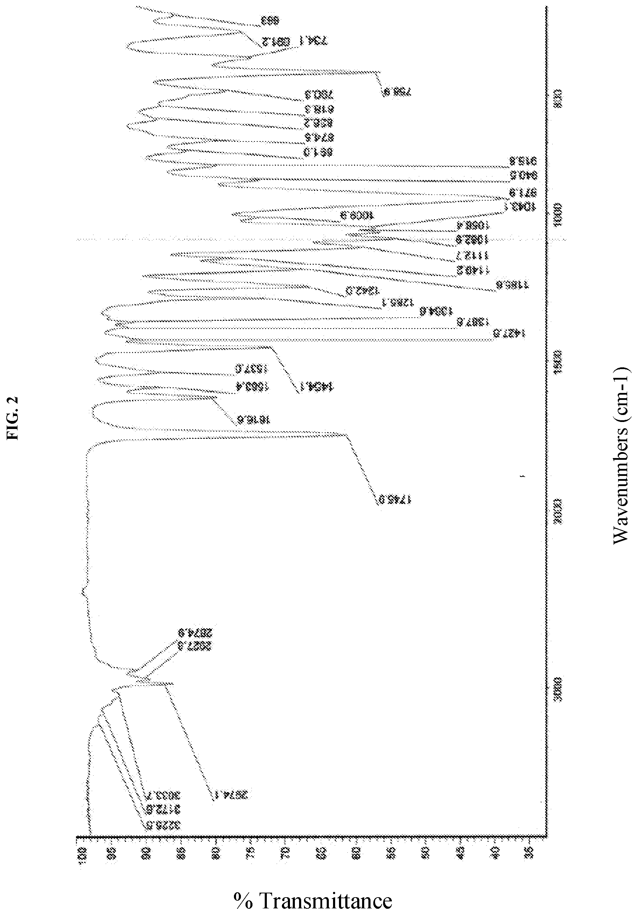

The Fourier Transformed-Infrared absorption (FT-IR) spectrum of Compound 1-ethanolate Form A was obtained using Attenuated Total Reflectance (ATR) on a Thermo-Nicolet Avatar 370 spectrophotometer using the neat material (See FIG. 2). The FT-IR spectrum band assignments are provided in Table 5. The results are in agreement with the chemical structure of Compound 1-ethanolate.

**Table 5. Characteristic infrared absorption bands (wavenumber) and the corresponding assignments for Compound 1-ethanolate Form A.**

| **Wavenumber (cm⁻¹)** | **Assignment** |
|---|---|
| 3034, 2974, 2928, 2875 | C-H |
| 1746 | C=O |
| 1617, 1583 | C=C |
| 1537 | NH |
| 1454 | CH₃, CH₂ |
| 1285, 1242, 1186, 1149, 1113, 1083, 1056 | C-O, C-N |
| 759, 691 | Aromatic H |

### Raman Spectroscopy

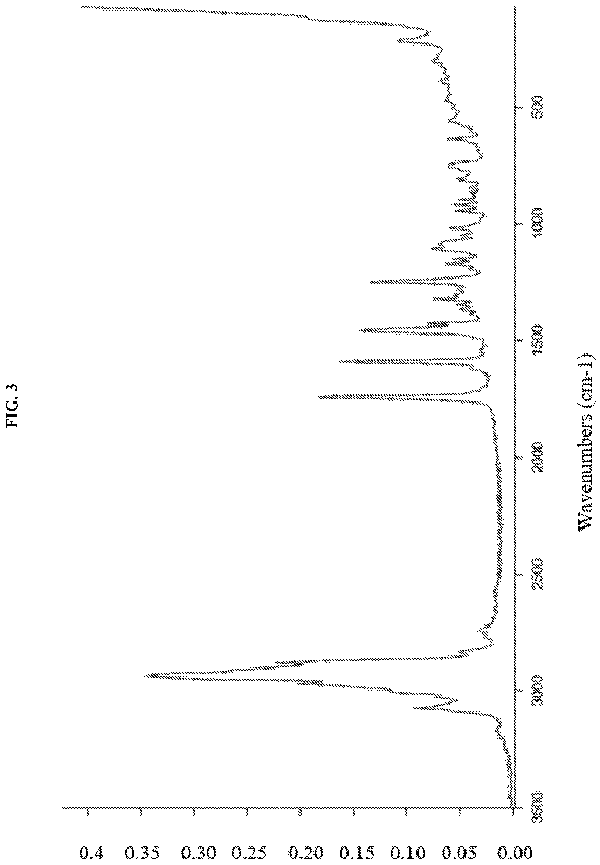

The FT-IR and Raman spectra for Compound 1 Form A was collected (See FIG. 3). The peaks are shown in the table below:

| **Peak** | **Wavenumber (cm⁻¹)** | **Peak** | **Wavenumber (cm⁻¹)** | **Peak** | **Wavenumber (cm⁻¹)** |
|---|---|---|---|---|---|
| 1 | 3075 | 11 | 1366 | 21 | 919 |
| 2 | 2969 | 12 | 1346 | 22 | 896 |
| 3 | 2936 | 13 | 1323 | 23 | 844 |
| 4 | 2879 | 14 | 1249 | 24 | 804 |
| 5 | 2836 | 15 | 1171 | 25 | 753 |
| 6 | 2745 | 16 | 1150 | 26 | 637 |
| 7 | 1743 | 17 | 1107 | 27 | 562 |
| 8 | 1589 | 18 | 1048 | 28 | 386 |
| 9 | 1457 | 19 | 1018 | 29 | 301 |
| 10 | 1429 | 20 | 944 | 30 | 216 |

### UV-Vis Spectroscopy

The Ultraviolet Absorbance Spectrum of Compound 1-ethanolate Form A was obtained on a PerkinElmer Lambda 25 UV-Vis spectrophotometer. The sample solution was prepared in methanol at 0.01 mg/mL. The spectrum absorption maxima with extinction coefficients are provided in Table 6.

**Table 6: Ultraviolet absorbance spectrum absorption maxima and extinction coefficient for Compound 1-ethanolate Form A.**

| **Solvent** | **Wavelength (nm)** | **Absorption** | **Molar Extinction Coefficient (L mol⁻¹ cm⁻¹)** |
|---|---|---|---|
| Methanol (0.01 mg/mL) | 208.3 | 0.66 | 27672 |
| | 238.5 | 0.19 | 7966 |
| | 302.3 | 0.10 | 4193 |

### Example 2. Solubility studies of Compound 1-ethanolate Form A.

Visual solubility estimates for Compound 1-ethanolate Form A were determined in a variety of solvents and solvent mixtures using an aliquot addition method to aid in experimental design. In general, Compound 1-ethanolate Form A exhibited good solubility in the majority of the tested solvents. Low solubility (<1 mg/mL) was observed in heptane and cyclohexane. Solubility results are provided in Table 7.

**Table 7: Solubility Estimates of Compound 1-ethanolate Form A at Ambient Temperature.**

| **Solvent^{a,b}** | **Solubility (mg/mL)^{c}** |
|---|---|
| Acetone | 34 |
| ACN | >66 |
| Anisole | 35 |
| Chloroform | 36 |
| Cyclohexane | <1 |
| DCM | 34 |
| Diethyl Ether | 9 |
| DMF | >63 |
| DMSO | >71 |
| EtOAc | >68 |
| EtOH | >68 |
| Heptane | <1 |
| IPA | 34 |
| IPOAc | >66 |
| MEK | 37 |
| MTBE | 22 |
| Nitromethane | >68 |
| TFE | >66 |
| THF | >64 |
| Toluene | 33 |
| Xylene | 17 |
| Cyclohexane:EtOAc 3:1 | 4 |
| Heptane:MEK 2:1 | 22 |
| Cyclohexane:EtOH 90:10 | 17 |

| | |
|---|---|
| a. Solvents were dried over 3Å molecular sieves prior to use, unless otherwise indicated. b. Solvent ratios are by volume. c. Solubilities are calculated based on the total solvent used to give a solution; actual solubilities may be greater because of the volume of the solvent portions utilized or a slow rate of dissolution. Solubilities are rounded to the nearest mg/mL. | |

### Example 3. XRPD characterization of Compound 1-ethanolate Form A.

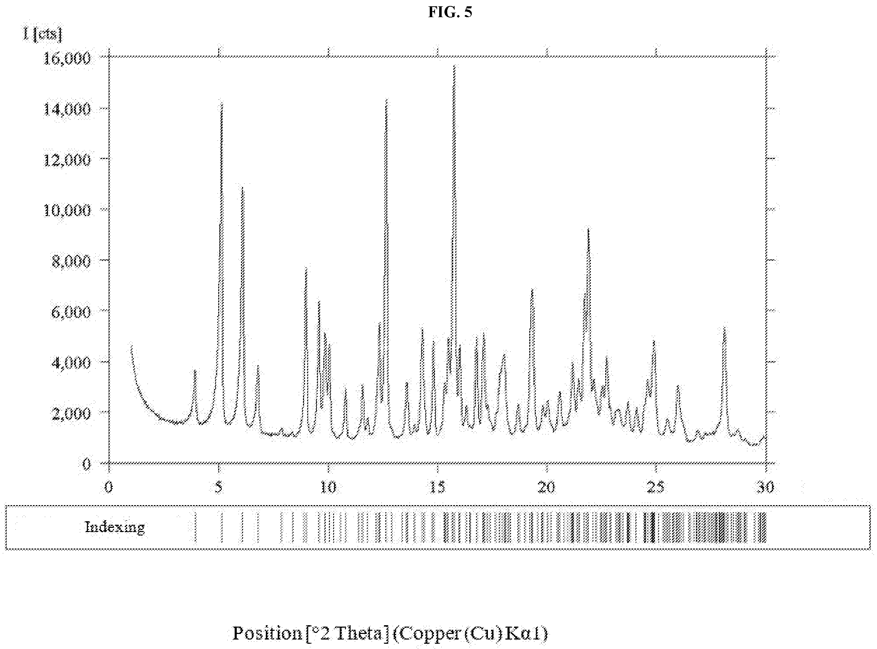

XRPD analysis indicates Compound 1-ethanolate Form A from Example 1 is composed of a crystalline material. FIG. 4 shows the XRPD pattern for Compound 1-ethanolate Form A. FIG. 5 shows the indexing solution for Compound 1-ethanolate Form A with Cu-Kα radiation.

The XRPD pattern of Compound 1-ethanolate Form A was successfully indexed, indicating that the sample is composed primarily or exclusively of a single crystalline phase.

| Bravais Type | Primitive Monoclinic |
|---|---|
| a [Å] | 17.377 |
| b [Å] | 11.945 |
| c [Å] | 22.629 |
| α [deg] | 90 |
| β [deg] | 96.51 |
| γ [deg] | 90 |
| Volume [Å³/cell] | 4666.7 |
| Chiral Contents? | Chiral |
| Extinction Symbol | P 1 2₁ 1 |
| Space Group(s) | P2₁ (4) |

### Example 4. Thermal Analysis of Compound 1-ethanolate Form A.

Thermal analysis of Compound 1-ethanolate Form A is presented in FIG. 6. The DSC thermogram of Compound 1-ethanolate Form A exhibits a small broad feature coincident with the onset of a single endotherm at 112.8 °C (peak maximum). The single endotherm is attributed to melting based on hot stage microscopy. A weight loss of 1.2% is observed in the TGA between 24.8 and 120 °C.

Hot stage microcopy of Compound 1-ethanolate Form A was conducted. Upon heating the sample, no changes were observed up to the onset of melting (i.e., 77.3 °C). Melting was completed at 106.5 °C and, upon cooling, no recrystallization of the melted sample was observed.

### Example 5: Dynamic Vapor Sorption (DVS) Analysis of Compound 1-ethanolate Form A.

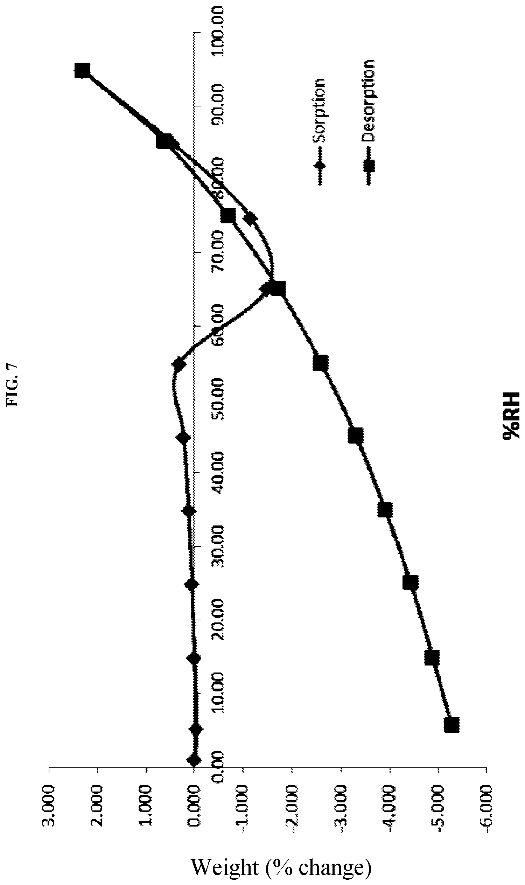

DVS analysis of Compound 1-ethanolate Form A was conducted from 5% relative humidity (RH) to 95% RH and back to 5% RH at 10% RH increments (FIG. 7). With increasing RH, the sample showed ~0.3 wt% gain between 5% RH and 55% RH suggesting Form A is non-hygroscopic from 5-55% RH. A loss of 1.8 wt% was observed between 55% RH and 65% RH possibly due to water displacing ethanol. After 65% RH, the sample gained 3.8 wt% between 65% RH and 95% RH. Notably, the sample mass did not meet the equilibrium criteria between 85% RH and 95% RH and all RH intervals during the desorption process. Weight loss (~7.6 wt%) was observed between 95% RH and 5% RH (i.e., desorption). After DVS analysis, it was noted that the sample had deliquesced.

Samples of Compound 1-ethanolate Form A were exposed to elevated RH (75%, 85% and 97%) at room temperature for approximately 3 hours and 24 hours (Table 8). After 3 hours, no deliquescence was observed at 75% RH, the sample appeared wet with solids present at 85% RH, and deliquescence was observed at 97% RH with minor birefringent solids present. After 24 hours, samples exposed to 85% RH and 97% RH deliquesced and the sample at 75% RH began deliquescing.

**Table 8. Time course of water absorption for Compound 1-ethanolate Form A under increasing relative humidities.**

| **Elap Time (min)** | **Weight (mg)** | **Weight % chg** | **Sample Temp °C** | **Sample RH %** |
|---|---|---|---|---|
| 0.1 | 14.448 | 0.000 | 25.18 | 0.99 |
| 21.1 | 14.441 | -0.043 | 25.19 | 5.15 |
| 39.7 | 14.447 | -0.003 | 25.19 | 14.86 |
| 51.8 | 14.455 | 0.051 | 25.20 | 24.90 |
| 64.8 | 14.465 | 0.120 | 25.19 | 34.83 |
| 78.3 | 14.479 | 0.214 | 25.19 | 44.87 |
| 91.2 | 14.493 | 0.316 | 25.18 | 54.86 |
| 281.1 | 14.232 | -1.494 | 25.18 | 65.03 |
| 294.7 | 14.279 | -1.170 | 25.20 | 74.65 |
| 356.0 | 14.513 | 0.451 | 25.19 | 84.85 |
| 543.7 | 14.782 | 2.311 | 25.19 | 94.83 |
| 731.2 | 14.539 | 0.633 | 25.19 | 85.19 |
| 918.9 | 14.347 | -0.694 | 25.19 | 74.98 |
| 1106.5 | 14.200 | -1.716 | 25.19 | 65.14 |
| 1296.4 | 14.073 | -2.592 | 25.19 | 54.99 |
| 1483.9 | 13.970 | -3.308 | 25.19 | 45.11 |
| 1671.4 | 13.882 | -3.916 | 25.19 | 35.03 |
| 1859.0 | 13.807 | -4.432 | 25.19 | 25.06 |
| 2044.7 | 13.742 | -4.883 | 25.19 | 14.87 |
| 2228.6 | 13.686 | -5.275 | 25.19 | 5.67 |

A separate DVS analysis using a stepped increase in relative humidity revealed that the mass increased by 0.3 wt.-% for Form A from 0% RH to 40% RH. At higher humidities, a decrease in mass was observed, due to loss of ethanol from the boronate ester complex. However, up to 60% r.h., the material retained its predominant crystal structure for at least four hours.

### Example 6. Mass Spectrometric Analyses of Compound 1-ethanolate Form A. Evidence for the conversion of Compound 1-ethanolate to ((2-ethylbutanoyl)oxy)methyl (R)-2-hydroxy-3-propionamido-3,4-dihydro-2H-benzo[e][1,2]oxaborinine-8-carboxylate (Reference Compound 1) in aqueous solution.

Mass spectrometric (MS) analyses were performed to confirm the structure of Compound 1-ethanolate Form A, and evaluate the ability of EtOH to dissociate from Compound 1-ethanolate in water. Compound 1-ethanolate Form A, and an amorphous preparation of ((2-ethylbutanoyl)oxy)methyl (*R*)-2-hydroxy-3-propionamido-3,4-dihydro-2*H*-benzo[*e*][1,2]oxaborinine-8-carboxylate were diluted in absolute EtOH or water and analyzed by high-resolution mass spectrometry. A sample of amorphous ((2-ethylbutanoyl)oxy)methyl (*R*)-2-hydroxy-3-propionamido-3,4-dihydro-2*H*-benzo[*e*][1,2]oxaborinine-8-carboxylate, dissolved in absolute EtOH, was spiked with water prior to analysis. The mass spectrum of Compound 1-ethanolate Form A diluted in absolute EtOH showed major peaks consistent with sodiated Compound 1-ethanolate (M+Na)⁺ at nominal 442 Da, and a sodium bound dimer of Compound 1-ethanolate (2M+Na)⁺ at nominal 861 Da. The exact mass observed for sodiated Compound 1-ethanolate was 442.2007 (difference = 1.4 ppm vs. theoretical: C₂₁H₃₀BNO₇Na⁺: 442.2013 Da).

The mass spectrum of Compound 1-ethanolate diluted in water showed major peaks at nominal 414 Da (M+Na)⁺, 787 Da (2M-H₂O+Na)⁺, 805 Da (2M+Na)⁺, 1178 Da (3M-H₂O+Na)⁺, and 1552 Da (4M-2H₂O +Na)⁺ corresponding to ((2-ethylbutanoyl)oxy)methyl (R)-2-hydroxy-3-propionamido-3,4-dihydro-2*H*-benzo[*e*][1,2]oxaborinine-8-carboxylate (Reference Compound 1).

The mass spectrum of ((2-ethylbutanoyl)oxy)methyl (*R*)-2-hydroxy-3-propionamido-3,4-dihydro-2*H*-benzo[*e*][1,2]oxaborinine-8-carboxylate, amorphous, diluted in absolute EtOH showed major peaks at nominal 442 Da and 861 Da, corresponding to sodiated Compound 1-ethanolate (M+Na)⁺ and a sodium bound dimer of Compound 1-ethanolate (2M+Na)⁺, respectively. This is the same result as observed for Compound 1-ethanolate when diluted in EtOH and is likely due to ethyl (boron) ester formation, i.e., azeotropic removal of water, during the electrospray ionization process.

The mass spectrum of ((2-ethylbutanoyl)oxy)methyl (*R*)-2-hydroxy-3-propionamido-3,4-dihydro-2*H*-benzo[*e*][1,2]oxaborinine-8-carboxylate, amorphous, dissolved in absolute EtOH then spiked with water shows major peaks at nominal 414 Da (M+Na)⁺, 787 Da (2M-H₂O+Na)⁺, 805 Da (2M+Na)⁺, 1178 Da (3M-H₂O+Na)⁺, and 1552 Da (4M-2H₂O +Na)⁺. These correspond to sodiated ((2-ethylbutanoyl)oxy)methyl (*R*)-2-hydroxy-3-propionamido-3,4-dihydro-2*H-*benzo[*e*][1,2]oxaborinine-8-carboxylate (M+Na)⁺, (2M-H₂O+Na)⁺, (2M+Na)⁺, (3M-H₂O+Na)⁺, and (4M-2H₂O +Na)⁺; respectively. These are the same results observed for the sample of Compound 1-ethanolate Form A diluted in water, suggesting that Compound 1-ethanolate was rapidly converted to the free boronic acid ((2-ethylbutanoyl)oxy)methyl (R)-2-hydroxy-3-propionamido-3,4-dihydro-2*H-*benzo[*e*][1,2]oxaborinine-8-carboxylate in the presence of water.

### Example 6. Preparation of Compound 1-ethanolate Form B.

A unique crystalline material, designated Form B, was observed from four experiments as mixtures with Compound 1-ethanolate Form A: 1) a heat/cool experiment of Compound 1-ethanolate in 5% EtOH in heptane; 2) stirring a solution that resulted from the addition of heptane to a solution of Compound 1-ethanolate in DCM; 3) stirring a hazy solution that resulted from the addition of cyclohexane to a solution produced after cooling a Compound 1-ethanolate solution in EtOAc:cyclohexane (1:3 v/v); and, 4) stirring a sample resulting from addition of heptane to an anisole solution containing Compound 1-ethanolate.

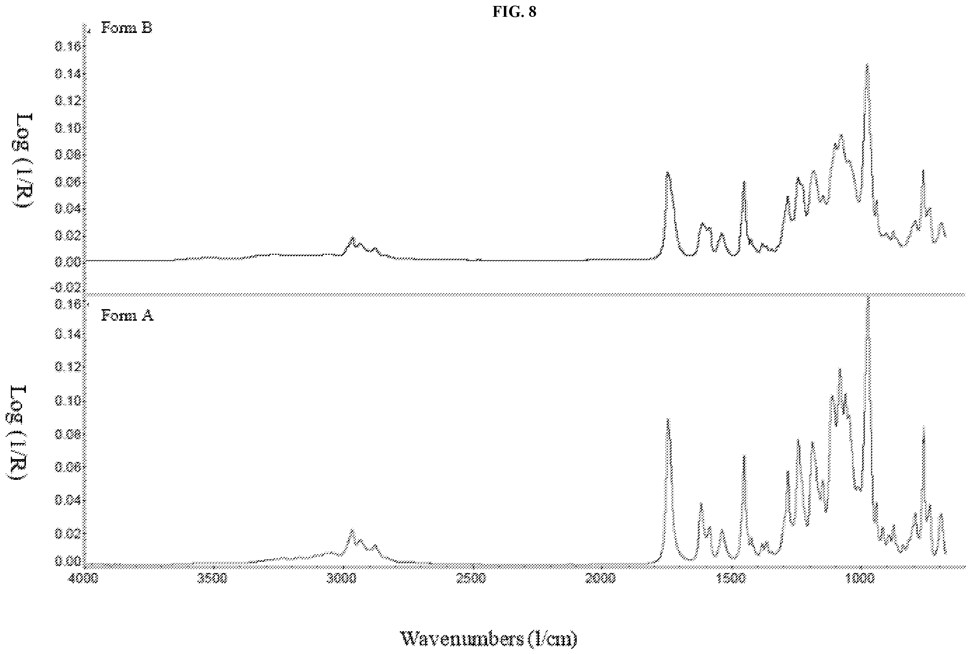

The infrared spectrum of Form B with minor Form A, was obtained. An overlay of the FT-IR spectra for Form B and Form A is shown in FIG. 8.

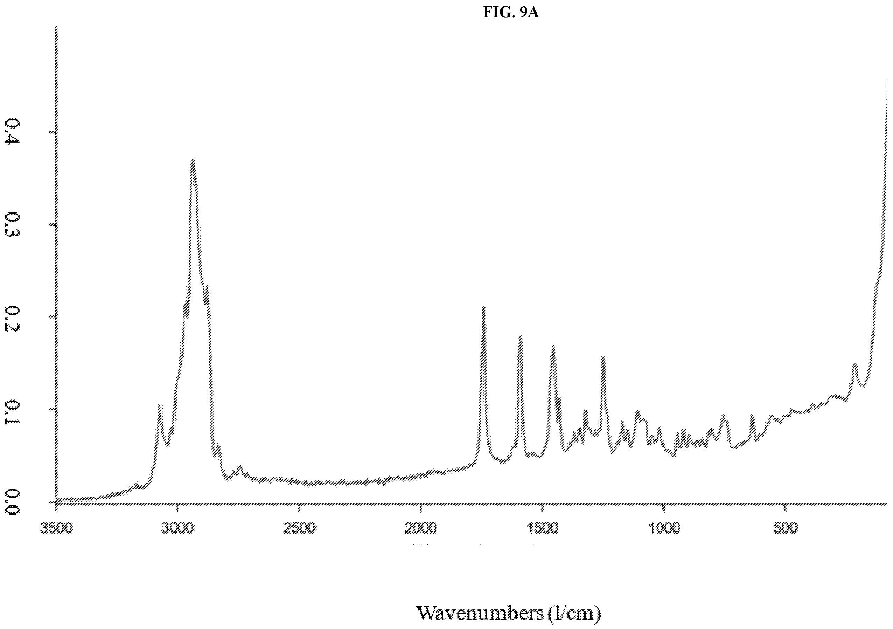

Compound 1-ethanolate Form B was further analyzed by Raman Spectroscopy, FIG. 9A. the peaks are shown in the table below:

| **Peak** | **Wavenumber (cm⁻¹)** | **Peak** | **Wavenumber (cm⁻¹)** | **Peak** | **Wavenumber (cm⁻¹)** |
|---|---|---|---|---|---|
| 1 | 3073 | 10 | 1429 | 19 | 944 |
| 2 | 2967 | 11 | 1367 | 20 | 918 |
| 3 | 2935 | 12 | 1345 | 21 | 895 |
| 4 | 2879 | 13 | 1322 | 22 | 844 |
| 5 | 2834 | 14 | 1249 | 23 | 805 |
| 6 | 2744 | 15 | 1171 | 24 | 753 |
| 7 | 1740 | 16 | 1150 | 25 | 636 |
| 8 | 1589 | 17 | 1108 | 26 | 556 |
| 9 | 1455 | 18 | 1018 | 27 | 217 |

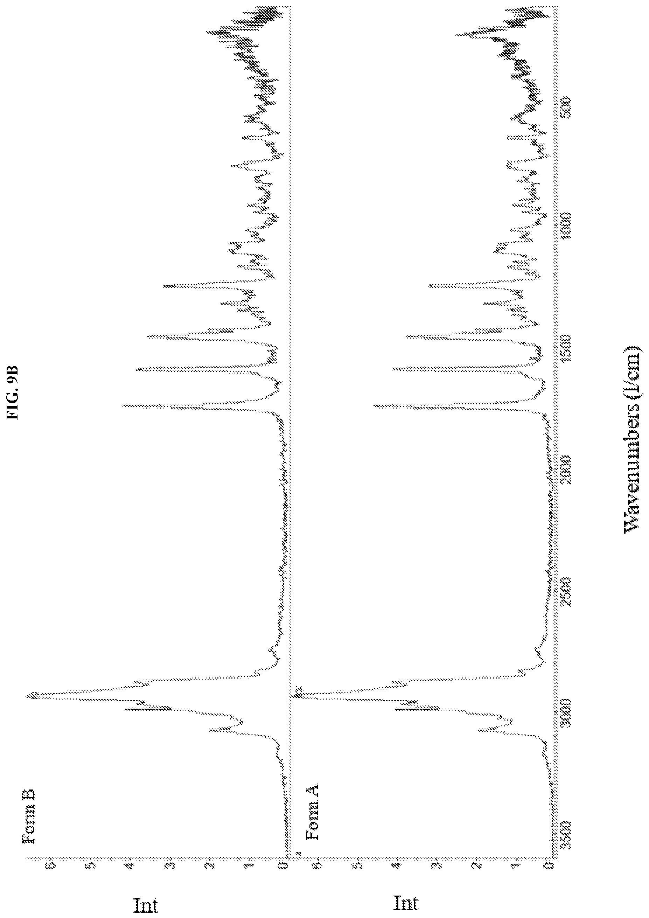

An overlay of the Raman spectra of Form B and Form A is presented in FIG. 9B.

### Example 7. XRPD characterization of Compound 1-ethanolate Form B.

The indexed XRPD pattern of Compound 1-ethanolate, Form B with minor Form A is illustrated in FIG. 10. Agreement between the allowed peak positions, marked with bars, and the observed peaks indicates a consistent unit cell determination. Successful indexing of the pattern indicates that the sample is composed primarily of a single crystalline phase. However, very minor peaks at 6.10°, 9.60°, 9.84°, and 10.05° are inconsistent with the allowed peaks for the indexed XRPD pattern of Form B thus suggesting the presence of an additional crystalline phase, specifically Form A.

| Bravais Type | Primitive Monoclinic |
|---|---|
| a [Å] | 11.958 |
| b [Å] | 11.383 |
| c [Å] | 17.329 |
| α [deg] | 90 |
| β [deg] | 92.40 |
| γ [deg] | 90 |
| Volume [Å³/cell] | 2356.8 |
| Chiral Contents? | Chiral |
| Extinction Symbol | P 1 2₁ 1 |
| Space Group(s) | P2₁ (4) |

### Example 8. Thermal analysis of Compound 1-ethanolate Form B.

Thermal analysis (DSC) revealed that Form B exhibited a small, broad feature at 77.7 °C (peak maximum) followed by an endotherm at 116.9 °C (peak maximum) which is likely attributable to melting (FIG. 11). A weight loss of 1.2% was observed between 23.6 °C and 125.0 °C by TGA analysis.

### Example 9: Dynamic Vapor Sorption (DVS) Analysis of Compound 1-ethanolate Form B.

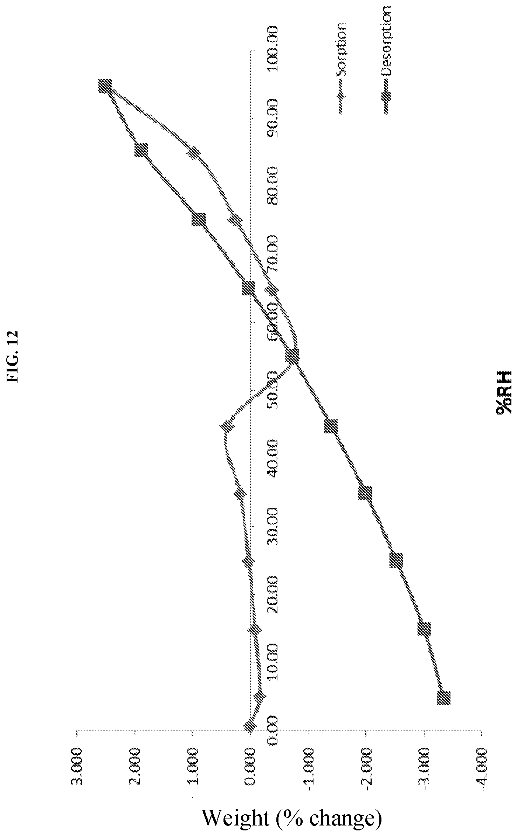

DVS analysis of Compound 1-ethanolate Form B (with minor Form A) was conducted from 5% RH to 95% RH and back to 5% RH at 10% RH increments (FIG. 12). The DVS isotherm of Form B is qualitatively similar to that of Form A. Form B lost 0.2 wt% upon equilibration at ~5% RH. With increasing RH, the sample showed ~0.5 wt% gain between 5% RH and 45% RH. A weight loss of 1.1% was observed between 45% RH and 55% RH, possibly due to the displacement of the ethanol with water. After 55% RH, the sample gained 3.3 wt% between 55% RH and 95% RH. The sample mass did not meet the equilibrium criteria between 85% RH and 95% RH and all RH intervals during the desorption process to 15% RH. Weight loss (~5.8 wt%) was observed during desorption from 95% RH to 5% RH. Following DVS analysis, it was noted that the sample appeared to have deliquesced.

A separate DVS analysis using a stepped increase in relative humidity revealed that the mass increased by 0.4 wt.-% for Form B from 0% RH to 40% RH. At higher humidities, a decrease in mass was observed, due to loss of ethanol from the boronate ester complex. However, up to 60% r.h., the material retained its predominant crystal structure for at least four hours.

### Example 10. Interconversion Slurries.

The difference in free energy between solid phases of the same composition (i.e. true polymorphs) is related to their relative solubilities, with the most stable polymorph having the lowest solubility in any solvent compared to a metastable polymorph. Therefore, a saturated solution with respect to the most stable form is undersaturated with respect to the less stable form. In the presence of seeds of different polymorphs, the less stable polymorph will therefore dissolve over time, resulting in further growth of the most stable form.

Solvent-mediated slurry interconversion experiments using seeds of different polymorphs were conducted to determine the most stable form of Compound 1-ethanolate. Saturated solutions of Compound 1-ethanolate were prepared in 3% DCM in heptane and 5% EtOH in heptane. Approximately equal amounts of Form A, and Form B (containing a minor amount of Form A) were added to the filtered saturated solutions, and the suspensions were slurried at room temperature for ~2 weeks, and the isolated solids were observed by polarized light microscopy (PLM) and analyzed by XRPD. XRPD patterns of the solids isolated from both slurries were consistent with Form A. These data suggest Form A is the stable form at room temperature.

### Reference Example 11: Preparation and Characterization of ((2-Ethylbutanoyl)oxy)methyl (R)-2-methoxy-3-propionamido-3,4-dihydro-2H-benzo[e][1,2]oxaborinine-8-carboxylate (Reference Compound 1-methanolate)

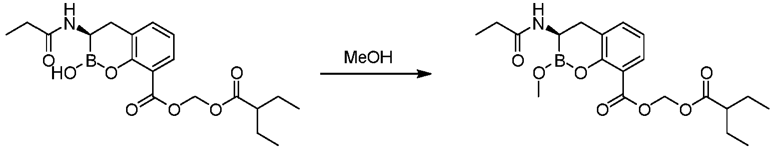

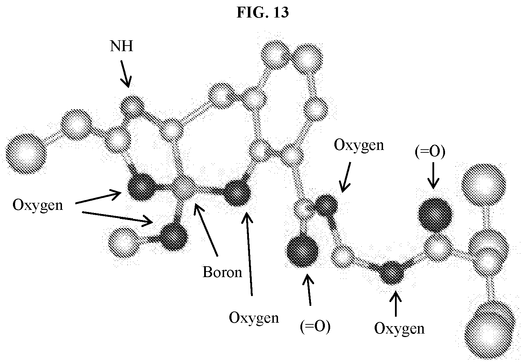

((2-Ethylbutanoyl)oxy)methyl (*R*)-2-hydroxy-3-propionamido-3,4-dihydro-2*H-*benzo[*e*][1,2]oxaborinine-8-carboxylate (Reference Compound 1) in methanol yielded ((2-Ethylbutanoyl)oxy)methyl (R)-2-methoxy-3-propionamido-3,4-dihydro-2H-benzo[e][1,2]oxaborinine-8-carboxylate (Reference Compound 1-methanolate). FIG. 13 shows the x-ray structure of Reference Compound 1-methanolate.

## Claims

1. A crystalline form of the compound: 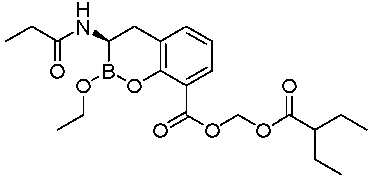 wherein:
the crystalline form has an X-ray powder diffraction (XRPD) pattern comprising characteristic peaks at 6.1 °±0.1° 2θ, 9.9 °±0.1° 2θ, and 16.0 °±0.1° 2θ.

2. The crystalline form of claim 1, wherein:
the X-ray powder diffraction (XRPD) pattern further comprises characteristic peaks at 19.3 °±0.1° 2θ, 6.8 °±0.1° 2θ, and 17.9 °±0.1° 2θ.

3. The crystalline form of claim 1 or 2, wherein:
the X-ray powder diffraction (XRPD) pattern further comprises characteristic peaks at 14.3 °±0.1° 2θ and 21.2 °±0.1° 2θ.

4. The crystalline form of claim 1, wherein:
the crystalline form has a DSC thermogram with a broad endotherm having an onset at 112.8 °C.

5. A crystalline form of the compound: 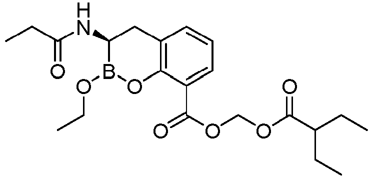 wherein:
the crystalline form has an X-ray powder diffraction (XRPD) pattern comprising characteristic peaks at 9.3 °±0.1° 2θ, 12.9 °±0.1° 2θ, and 21.5 °±0.1° 2θ.

6. The crystalline form of claim 5, wherein:
the X-ray powder diffraction (XRPD) pattern further comprises characteristic peaks at 8.8 °±0.1° 2θ, 14.6 °±0.1° 2θ, and 17.3 °±0.1° 2θ.

7. The crystalline form of claim 5, wherein:
the crystalline form has a DSC thermogram with a broad endotherm having an onset at 116.9 °C.

8. A crystalline form of any one of claims 1-7 for use in combination with a beta-lactam antibiotic in a method of treating a bacterial infection in a subject.

9. The crystalline form for use according to claim 8, wherein:
the beta-lactam antibiotic is ceftibuten, or a salt thereof.

10. A pharmaceutical composition comprising:
(a) a crystalline form of any one of claims 1-7; and
(b) a pharmaceutically acceptable excipient.

11. The pharmaceutical composition of claim 10, further comprising:
a beta-lactam antibiotic.

12. The pharmaceutical composition of claim 11, wherein:
the beta-lactam antibiotic is ceftibuten, or a salt thereof.

13. A pharmaceutical composition of any one of claims 10-12 for use in a method of treating a bacterial infection in a subject.

## Patentansprüche

1. Kristalline Form der Verbindung: 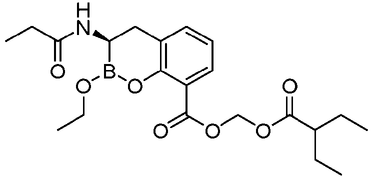 wobei:
die kristalline Form ein Röntgenpulverbeugungsmuster (XRPD-Muster) mit charakteristischen Peaks bei 6,1°±0,1° 2θ, 9,9°±0,1° 2θ und 16,0°±0,1° 2θ aufweist.

2. Kristalline Form nach Anspruch 1, wobei:
das Röntgenpulverbeugungsmuster (XRPD-Muster) ferner charakteristische Peaks bei 19,3°±0,1° 2θ, 6,8°±0,1° 2θ und 17,9°±0,1° 2θ umfasst.

3. Kristalline Form nach Anspruch 1 oder 2, wobei:
das Röntgenpulverbeugungsmuster (XRPD-Muster) ferner charakteristische Peaks bei 14,3°±0,1° 2θ und 21,2°±0,1° 2θ umfasst.

4. Kristalline Form nach Anspruch 1, wobei:
die kristalline Form ein DSC-Thermogramm mit einer breiten Endotherme mit einem Beginn bei 112,8 °C aufweist.

5. Kristalline Form der Verbindung: 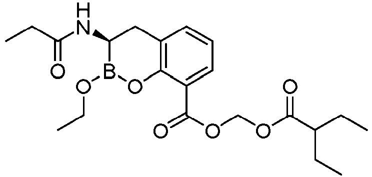 wobei:
die kristalline Form ein Röntgenpulverbeugungsmuster (XRPD-Muster) mit charakteristischen Peaks bei 9,3°±0,1° 2θ, 12,9°±0,1° 2θ und 21,5°±0,1° 2θ aufweist.

6. Kristalline Form nach Anspruch 5, wobei:
das Röntgenpulverbeugungsmuster (XRPD-Muster) ferner charakteristische Peaks bei 8,8°±0,1° 2θ, 14,6°±0,1° 2θ und 17,3°±0,1° 2θ umfasst.

7. Kristalline Form nach Anspruch 5, wobei:
die kristalline Form ein DSC-Thermogramm mit einer breiten Endotherme mit einem Beginn bei 116,9 °C aufweist.

8. Kristalline Form nach einem der Ansprüche 1-7 zur Verwendung in Kombination mit einem Beta-Lactam-Antibiotikum bei einem Verfahren zur Behandlung einer bakteriellen Infektion bei einem Individuum.

9. Kristalline Form zur Verwendung nach Anspruch 8, wobei:
es sich bei dem Beta-Lactam-Antibiotikum um Ceftibuten oder ein Salz davon handelt.

10. Pharmazeutische Zusammensetzung, umfassend:
(a) eine kristalline Form nach einem der Ansprüche 1-7; und
(b) einen pharmazeutisch unbedenklichen Hilfsstoff.

11. Pharmazeutische Zusammensetzung nach Anspruch 10, ferner umfassend:
ein Beta-Lactam-Antibiotikum.

12. Pharmazeutische Zusammensetzung nach Anspruch 11, wobei:
es sich bei dem Beta-Lactam-Antibiotikum um Ceftibuten oder ein Salz davon handelt.

13. Pharmazeutische Zusammensetzung nach einem der Ansprüche 10-12 zur Verwendung bei einem Verfahren zur Behandlung einer bakteriellen Infektion bei einem Individuum.

## Revendications

1. Forme cristalline du composé : 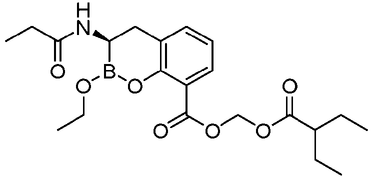 dans laquelle :
la forme cristalline a un diagramme de diffraction des rayons X sur poudre (XRPD) comprenant des pics caractéristiques à 6,1 °±0,1° 20, 9,9 °±0,1° 20, et 16,0 °±0,1° 2θ.

2. Forme cristalline de la revendication 1, dans laquelle :
le diagramme de diffraction des rayons X sur poudre (XRPD) comprend en outre des pics caractéristiques à 19,3 °±0,1 ° 2θ, 6,8 °±0,1° 2θ, et 17,9 °±0,1° 2θ.

3. Forme cristalline de la revendication 1 ou 2, dans laquelle :
le diagramme de diffraction des rayons X sur poudre (XRPD) comprend en outre des pics caractéristiques à 14,3 °±0,1° 2θ et 21,2 °±0,1° 2θ.

4. Forme cristalline de la revendication 1, dans laquelle :
la forme cristalline présente un thermogramme de DSC avec un endotherme large ayant un début à 112,8 °C.

5. Forme cristalline du composé : 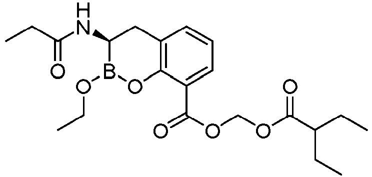 dans laquelle :
la forme cristalline a un diagramme de diffraction des rayons X sur poudre (XRPD) comprenant des pics caractéristiques à 9,3 °±0,1° 2θ, 12,9 °±0,1° 2θ, et 21,5 °±0,1° 2θ.

6. Forme cristalline de la revendication 5, dans laquelle :
le diagramme de diffraction des rayons X sur poudre (XRPD) comprend en outre des pics caractéristiques à 8,8 °±0,1° 2θ, 14,6 °±0,1° 2θ, et 17,3 °±0,1° 2θ.

7. Forme cristalline de la revendication 5, dans laquelle :
la forme cristalline présente un thermogramme de DSC avec un endotherme large ayant un début à 116,9 °C.

8. Forme cristalline de l'une quelconque des revendications 1 à 7 pour utilisation en combinaison avec un antibiotique de type bêta-lactame dans un procédé de traitement d'une infection bactérienne chez un sujet.

9. Forme cristalline pour une utilisation selon la revendication 8, dans laquelle :
l'antibiotique de type bêta-lactame est le ceftibuten, ou un sel de celui-ci.

10. Composition pharmaceutique comprenant :
(a) une forme cristalline de l'une quelconque des revendications 1 à 7 ; et
(b) un excipient pharmaceutiquement acceptable.

11. Composition pharmaceutique de la revendication 10, comprenant en outre :
un antibiotique de type bêta-lactame.

12. Composition pharmaceutique de la revendication 11 dans laquelle :
l'antibiotique de type bêta-lactame est le ceftibuten, ou un sel de celui-ci.

13. Composition pharmaceutique de l'une quelconque des revendications 10 à 12 pour une utilisation dans un procédé de traitement d'une infection bactérienne chez un sujet.
